# EUROPEAN PATENT APPLICATION

(11) **EP 0 574 607 A1**
(43) Date of publication of application: **22.12.1993**
(21) Application number: 92110290.1
(22) Date of filing: 17.06.1992
(51) Int. Cl.: A61K 7/06, A61K 7/11

(54) **Aqueous aerosol hair spray**

(30) Priority: 18.06.1992 US 716708
(71) Applicant: HELENE CURTIS, INC., Chicago, Illinois 60610-4713 (US)
(72) Inventor: Galleguillos, Ramiro, Glendale Heights, Ill. 60139 (US)
(74) Representative: Patentanwälte Dipl.-Ing. R. Splanemann Dr. B. Reitzner Dipl.-Ing. K. Baronetzky

(57) **Abstract**

An aqueous aerosol hair styling aid or mousse composition, and method, including a sulfonated polystyrene homopolymer or copolymer fixative resin in an amount of about 2% to about 20% by weight of the composition, particularly about 2% to about 15% by weight of the composition; water in an amount of about 30% to about 90% by weight of the composition; alcohol in an amount of 0% to about 30% by weight; and a water soluble liquifable propellant gas, such as dimethyl ether, in an amount of about 5% to about 50% based on the total weight of the aerosol composition.

## Description

### FIELD OF THE INVENTION

The present invention is directed to an aerosol aqueous hair styling aid that is sprayed onto the hair from an aqueous composition, preferably an aerosol aqueous composition containing a propellant, to provide the hair with a particular shape or configuration. More particularly, the present invention is directed to an aqueous aerosol hair styling aid that has a very low percentage of volatile organic compounds and a high percentage of water, and is capable of homogeneously dispersing a relatively high percentage of a sulfonated partially or fully neutralized polymer of styrene and/or a sulfonated copolymer of styrene and a copolymerizable ethylenically unsaturated comonomer, with or without a water evaporation agent, such as an alcohol, for example, ethanol.

### BACKGROUND OF THE INVENTION AND PRIOR ART

Hair sprays provide human hair with a particular shape or configuration and function by applying a thin film of a resin or gum onto the hair to adhere adjacent hairs together so that they retain the particular shape or configuration at the time of application. Many of these hair sprays have been applied from aerosol compositions that include a liquifiable propellant gas - generally a halohydrocarbon, such as trichlorofluoromethane or trichlorotrifluoroethane or a gaseous hydrocarbon such as propane or butane. Recently-proposed legislation directed to the depletion of the atmospheric ozone layer has led to the halohydrocarbons being increasingly replaced with pure gaseous non-halogenated hydrocarbons as propellants. However, the use of non-halogenated hydrocarbons as propellants has resulted in a problem of decreased solubility of the hair spray resin or gum in water requiring an increased amount of a volatile organic solvent, such as ethanol, to achieve sufficient solubility, therefore creating an additional ecological problem.

As set forth in the Nuber et al. U.S. Patent No. 4,767,613, film-forming polymers based on carboxyl-containing resins can be at least partially neutralized to improve the water solubility or dispersibility of the resin and provide the resin with the quality of being easier to remove from the hair during washing. However, as disclosed in the Nuber et al. U.S. Patent No. 4,767,613, such aerosol hair treating agents generally have a maximum of about 5% water and require a high amount, up to about 95% by weight, of a volatile organic solvent, such as alcohol, despite the increased solubility of the neutralized resin.

Turbek U.S. Patent No. 3,072,618, hereby incorporated by reference, describes the synthesis and preparation of the sulfonated polymers useful in accordance with the present invention. Further, Nowak, et al. U.S. Patent No. 3,972,336, hereby incorporated by reference, describes the use of these sulfonated styrene polymers and copolymers as hair fixatives applied from aerosol or non-aerosol compositions using water-immiscible compressed hydrocarbon propellant gases, such as isobutane and propane.

Stepan Company of Northfield, Illinois discloses one example of an aerosol hair spray that includes 41.10% by weight water, but the aerosol composition also includes 45.70% by weight alcohol, as follows, where percentages are present by weight: Terpolymer of polyvinyl pyrrolidone, ethylmethacylate, and methacrylic acid 9.60%; SDA 40 Alcohol (190 Proof) 45.70%; 2-methyl-2-amino-1-propanol (AMP) 0.40%; Silicone L-722 (Union Carbide) 0.50%; Propylene Glycol 2.00%; Perfume V-5177 (Van Dyke) 0.60%; 5% Morpholine in SDA 40 Alcohol 0.10%; and Distilled Water 41.10%.

In accordance with the present invention, a new and improved aqueous aerosol hair styling aid composition has been found wherein partially or fully neutralized sulfonated polymers and copolymers having a weight average molecular weight in the range of about 55,000 to about 1,000,000 can be suitably solubilized in the aqueous composition containing about 30% to about 90% by weight water and less than 20% by weight volatile organic compounds, particularly less than about 35% volatile organic compounds, e.g., 0% to about 30% ethanol, preferably about 5% to about 25% alcohol, while providing an aerosol styling aid or mousse that can effectively solubilize up to about 20% sulfonated polystyrene polymers and copolymers, with a volatile organic solvent being optional for faster drying.

### SUMMARY OF THE INVENTION

In brief, the present invention is directed to an aerosol aqueous hair styling aid or mousse composition, and method, including a water-soluble or water-dispersable sulfonated polymer and/or copolymer in an amount of about 2% to about 20% by weight of the composition, particularly about 2% to about 15% by weight of the composition; water in an amount of about 30% to about 90% by weight of the composition; alcohol in an amount of 0% to about 30% by weight; and a liquified propellant gas, such as dimethyl ether, in an amount of about 5% to about 50% based on the total weight of the aerosol composition. The sulfonate moieties of the fixative polymer or copolymer should be neutralized about 30% to about 100% by weight to achieve sufficient water solubility.

Accordingly, one aspect of the present invention is to provide a new and improved hair treating composition, and method, that can be applied to the hair from an aqueous aerosol composition in the form of a hair spray, mousse, or foam that provides hair setting compositions for retaining a particular shape or configuration of the hair.

Another aspect of the present invention is to provide an aerosol hair styling aid composition, and method, in the form of a hair spray, mousse, or foam that includes water in an amount of about 30% to about 90% by weight of the composition, a fixative sulfonated styrene polymer and/or copolymer resin that is solubilized or dispersed in an amount of about 2% to about 20% by weight of the composition, and a dimethyl ether propellant gas.

Another aspect of the present invention is to provide an aerosol hair styling aid composition, and method, in the form of a hair spray, mousse, or foam, that includes at least 30% by weight water, a partially or fully neutralized sulfonated styrene polymer or copolymer fixative resin and a dimethyl ether propellant.

Still another aspect of the present invention is to provide a new and improved aerosol hair styling aid composition that includes water in an amount of about 30% to about 90% by weight of the composition, a sulfonated styrene polymer or copolymer fixative resin that is solubilized or dispersed in an amount of about 2% to about 20% by weight of the composition, alcohol in an amount of 0% to about 30%, preferably about 5% to about 25% by weight, and a dimethyl ether propellant gas.

A further aspect of the present invention is to provide a new and improved aerosol hair styling aid composition that includes water in an amount of about 30% to about 90% by weight of the composition, a sulfonated styrene polymer or copolymer fixative resin that is solubilized or dispersed in an amount of about 2% to about 20% by weight of the composition, alcohol in an amount of about 0% to about 30%, preferably about 5% to about 25%, by weight, a dimethyl ether propellant gas, and a conditioning agent in an amount of about 0.1% to about 10% by weight for mousses, or in an amount of about 0.1% to about 2% of the composition for hair sprays.

### BRIEF DESCRIPTION OF THE DRAWING

Figure 1 is a graph showing the phase diagram of a water-alcohol solution containing 3% by weight sodium polystyrene sulfonate having a weight average molecular weight of 130,000.

Figure 2 is a graph showing the phase diagram of a water-alcohol solution containing 6% by weight sodium polystyrene sulfonate having a weight average molecular weight of 130,000.

### DETAILED DESCRIPTION OF THE PREFERRED EMBODIMENT

Any of the following sulfonated styrene homopolymers and copolymers that are soluble or dispersible in the aqueous phase may be used; if an optional co-solvent such as ethanol is present, the polymer should be soluble or dispersible in the combined solvent system. Solubility or dispersibility is determined at ambient conditions (e.g., a temperature of about 25°C. and atmospheric pressure).

The polymers or resins useful in the compositions of the present invention are partially or fully neutralized sulfonated polystyrene homopolymers or copolymers that can be rendered dispersible or soluble in aqueous or hydroalcoholic solvent mixtures. Suitable polymers are disclosed in the Turbak U.S. Patent No. 3,072,618 and Nowak, Jr. U.S. Patent No. 3,972,336. The sodium polystyrene sulfonate fixative resins are preferred and have the general structure: 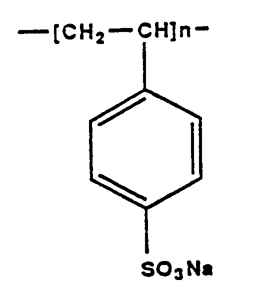
where n is typically between 250 (Mw ≃ 55,500) to 4,500 (Mw ≃ 1,000,000). Polymers with Mw values between 80,000 and 200,000 are preferred. Polymers with very low Mw tend to be brittle and do not have good film forming properties, hence they do not hold hair properly. Polymers with high Mw give viscous solutions in water and are more difficult to spray aesthetically. The final hair spray can contain between about 0.1% and about 8% by weight polymer.

If the fixative resin is a copolymer, the copolymer preferably includes from about 5% to about 45% by weight of an ethylenically unsaturated vinyl type comonomer capable of being copolymerized with styrene, such as the acrylic and methacrylic esters of aliphatic alcohols such as methyl, ethyl, butyl, and 2-ethyl hexyl alcohols; acrylic acid; acrylonitrile; methacrylonitrile; dibutyl maleate; vinylidene chloride; N-vinyl pyrrolidone; and the like. Any of these and other monomers which possess the characteristic, functional -CH=CH- group which copolymerizes with the styrene, yet is otherwise inert or at least will not appreciably interfere with subsequent sulfonation of the styrene units, may be used to make the interpolymers with styrene contemplated in this invention. Preferred interpolymers will be prepared using styrene in a proportion of at least 50 percent by weight.

Neutralization and increased solubilization are accomplished with one or more inorganic bases such as sodium hydroxide, potassium hydroxide, ammonium hydroxide and/or ammonium carbonate. Among stable organic bases are the water soluble bases such as monoethanolamine (MEA), diethanolamine (DEA), triethanolamine (TEA), 2-methyl-2-amino-1-propanol (AMP), monoamino glycols, and the like, which help solubilize the polymer in water solutions. The level of neutralization required for solubilization varies for each polymer. All of the above-described polymers and copolymers become soluble in water and hydroalcoholic solutions at 100% neutralization, and all described levels of water/alcohol/propellant solutions. The pH of these solutions usually ranges from 9 to 12. The lowest neutralization level needed to render the polymer water soluble or dispersible depends on the kind of polymer, and the amount of alcohol, water, and propellant. For instance, for a sodium polystyrene sulfonate homopolymer in water the lowest neutralization level is about 20% with sodium hydroxide and AMP. At neutralization levels of about 65% to about 75%, the pH of the solutions range from about 5 to about 7. A neutral pH such as this is preferred, however, the pH of the compositions of the present invention can vary from about 4 to about 13.

The solvent can be water or a water/alcohol, e.g., ethanol mixture. However large volumes of ethanol have a negative effect in the formulation. Aqueous sodium polystyrene sulfonate solutions cannot tolerate more than about 20% by weight ethanol. Further, the propellant gas miscibility decreases as the level of ethanol and resin increases, and while some ethanol can be allowed in the formulations, this usually results in large aerosol droplet size and/or foaming. For this reason it is preferred that the formulations contain no more than about 5% by weight alcohol which is sufficient to depress the freezing point of the formulation down to about -15°C.

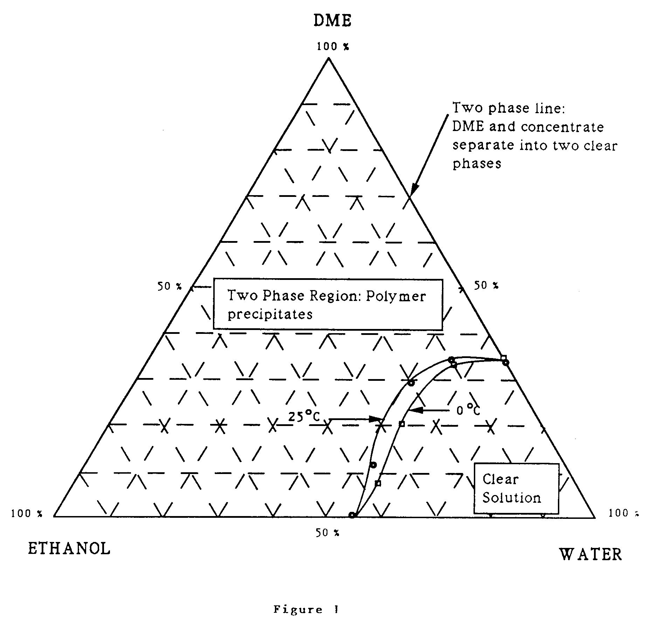

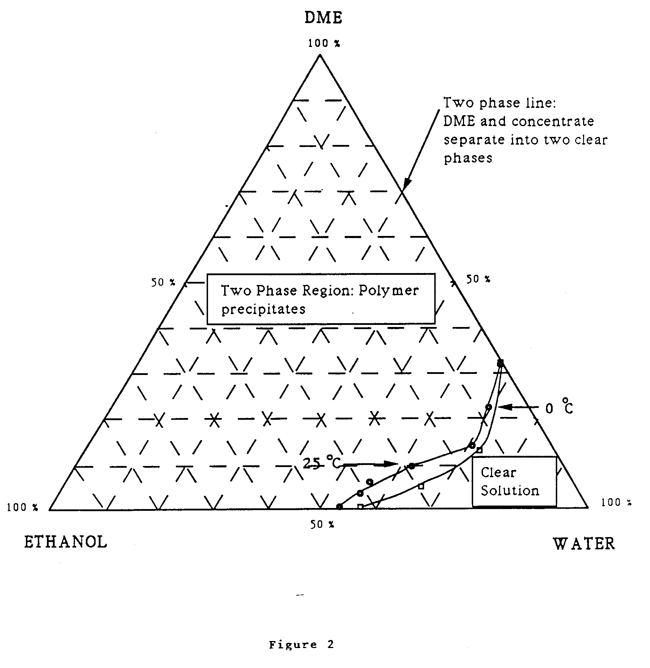

The preferred propellant is dimethyl ether (DME) since DME is soluble in water up to about 35% by weight. As shown in the phase diagrams of Figures 1 and 2, the amount of DME that can be incorporated into the compositions while maintaining clear solutions decreases rapidly as the level of ethanol and polymer increases. Therefore the amount of DME that can be incorporated depends on the amount of polymer and ethanol in the compositions. Other water-soluble gases such as carbon dioxide, and/or nitrous oxide can be blended with DME to obtain aerosols having reduced flammability.

A typical hair spray formulation according to the present invention is as follows:

| | |
|---|---|
| Resin: | 0.1 to 10% by weight |
| Ethanol: | 0 to 5% by weight |
| Water: | 50 to 80% by weight |
| DME: | 15 to 50% by weight |
| Coadjuvants: | 0 to 5% by weight |

Coadjuvants are chemical compounds such as fragrance, dye, preservatives, humectants, UV stabilizers, corrosion inhibitors, pH adjusters, and the like. Since these materials are added in small quantities they practically do not affect the phase diagrams. Although the solubility limit of DME in water formulations is about 35% by weight, larger quantities of DME than 35% can be used because any excess DME forms a separate phase sitting on top of the aqueous phase without effecting the performance of the composition.

The sulfonated styrene fixative polymer(s) is used at a level of from about 0.25% to about 20% by weight, generally about 2% to about 15% by weight, and preferably from about 1% to about 8% by weight of the total composition. The weight average molecular weight of the polymer is not critical but is generally in the range of from about 55,000 to about 1,000,000.

In accordance with one important embodiment of the present invention, the composition of the present invention also includes from about 0.1% to about 2% by weight, particularly about 0.5% to about 10% by weight for mousse compositions, of a non-volatile silicone compound or other conditioning agent(s), preferably a water-insoluble, emulsifiable conditioning agent. The preferred non-volatile silicone compound is a polydimethylsiloxane compound, such as a mixture, in about a 3:1 weight ratio, of a low molecular weight polydimethylsiloxane fluid and a higher molecular weight polydimethylsiloxane gum. The non-volatile polydimethylsiloxane compound is added to the composition of the present invention in an amount sufficient to provide improved combing and improved feel (softness) to the hair after shampooing. As referred to herein, "silicone gums" are those nonfunctional siloxanes having a viscosity of from about 5 to about 600,000 centistokes at 25°C. The so-called rigid silicones, as described in U.S. Patent No. 4,902,499, herein incorporated by reference, having a viscosity above 600,000 centistokes at 20°C, e.g. 700,000 centistokes plus, and a weight average molecular weight of at least about 500,000 also are useful in accordance with the present invention.

Preferred silicone gums include linear and branched polydimethylsiloxanes, of the following general formula:

(CH₃)₃SiO-[Si(CH₃)₂O]ₙ-Si(CH₃)₃ ,

wherein n is from about 2,000 to about 15,000, preferably from about 2,000 to about 7,000. Silicone gums useful in compositions of the present invention are available from a variety of commercial sources, including General Electric Company and Dow Corning.

Another particularly suitable conditioning agent that can be included in the composition of the present invention is a volatile hydrocarbon, such as a hydrocarbon including from about 10 to about 30 carbon atoms, that has sufficient volatility to slowly volatilize from the hair after application of the aerosol or non-aerosol styling aid composition. The volatile hydrocarbons provide essentially the same benefits as the silicone conditioning agents.

The preferred volatile hydrocarbon compound is an aliphatic hydrocarbon including from about 12 to about 24 carbon atoms, and having a boiling point in the range of from about 100°C to about 300°C. Exemplary volatile hydrocarbons are depicted in general structural formula (I), wherein n ranges from 2 to 5, 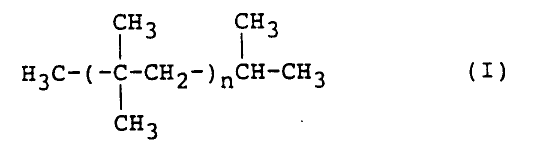
Examples of volatile hydrocarbons useful in the composition of the present invention are the commercially-available compounds PERMETHYL 99A and PERMETHYL 101A, corresponding to compounds of general structure (I) wherein n is 2 and 3, respectively, available from Permethyl Corporation, Frazer, PA. A volatile hydrocarbon compound is useful in the composition of the present invention either alone, in combination with another volatile hydrocarbon, or in combination with a volatile silicone.

Examples of other suitable water-insoluble conditioning agents that can be incorporated into the aerosol or non-aerosol aqueous styling aid composition of the present invention include the following: polysiloxane polyether copolymers; acetylated lanolin alcohols; lauryl dimethylamine oxide; a lanolin-derived extract of sterol on sterol esters; lanolin alcohol concentrate; an isopropyl ester of lanolin fatty acids; sulfur rich amino acid concentrates; isopropyl ester of lanolin fatty acids; oleyl alcohol; stearyl alcohol; a polyol fatty acid; a fatty amidoamine; cetyl/stearyl alcohol; keratin protein derivatives; isostearamidopropyl dimethylamine; stearamidopropyl dimethylamine; an amino-functional silicone; isopropyl ester of lanolic acids; ethoxylated (30) castor oil; acetylated lanolin alcohol; fatty alcohol fraction of lanolin; a mineral oil and lanolin alcohol mixture; high molecular weight esters of lanolin; vinylpyrrolidone/dimethylaminoethylmethacrylate copolymer; 5 mole ethylene oxide adduct of soya sterol; 10 mole ethylene oxide adduct of soya sterol; stearic acid ester of ethoxylated (20 mole) methyl glucoside; sodium salt of poly-hydroxycarboxylic acid; hydroxylated lanolin; ethylene glycol monostearate and propylene glycol mixture; acetamide MEA; lactamide MEA; stearamide MEA; cetearyl alcohol; mixed ethoxylated and propoxylated long chain alcohols; polonitomine oxide; oleamine oxide; stearamine oxide; hydrolyzed animal keratin; ethyl hydrolyzed animal keratin; stearamidoethyl diethylamine; cocamidopropyl dimethylamine; lauramidopropyl dimethylamine; oleamidopropyl dimethylamine; palmitamidopropyl dimethylamine; avocado oil; sweet almond oil, grape seed oil; jojoba oil; apricot kernel oil; sesame oil; hybrid safflower oil; wheat germ oil; behenamidopropyl betaine; ricinoleamidopropyl betaine; wheat germamidopropyl dimethylamine oxide; disodium isostearaimido MEA sulfosuccinate; disodium oleamide PEG-2 sulfosuccinate; disodium oleamide MEA sulfosuccinate; disodium ricinoleyl MEA sulfosuccinate; disodium wheat germamido MEA sulfosuccinate; disodium wheat germamido PEG-2 sulfosuccinate; stearamido amine; stearamido morpholine; isostearamido amine; isostearamido morpholine; polyethylene glycol (400) mono and distearates; synthetic calcium silicate; isostearic alkanolamide; ethyl esters of hydrolyzed animal protein; blend of cetyl and stearyl alcohols with ethoxylated cetyl or stearyl alcohols; amido amines; polyamido amines; palmityl amido betaine; propoxylated (1-20 moles) lanolin alcohols; isostearamide DEA; and hydrolyzed collagen protein.

When one or more of these water-insoluble conditioning agents is included in a mousse composition of the present invention in an amount of about 0.5% to about 10% by total weight of the composition, the composition also can include a suspending agent for the conditioning agent, in an amount of about 0.5% to about 10%, by total weight of the composition. The particular suspending agent is not critical and can be selected from any materials known to suspend water-insoluble liquids in shampoo compositions. Suitable suspending agents are for example, distearyl amate (distearyl phthalamic acid); fatty acid alkanolamides; esters of polyols and sugars; polyethylene glycols; the ethoxylated or propoxylated alkylphenols; ethoxylated or propoxylated fatty alcohols; and the condensation products of ethylene oxide with long chain amides. These suspending agents, as well as numerous others not cited herein, are well known in the art and are fully described in the literature, such as McCUTCHEON'S DETERGENTS AND EMULSIFIERS, 1989 Annual, published by McCutcheon Division, MC Publishing Co.

A nonionic alkanolamide also is optionally included in an amount of about 0.1% to about 5% by weight in the aerosol styling aid compositions, particularly for mousse compositions that include a conditioning agent, to provide exceptionally stable emulsification of water-insoluble conditioning agents and to aid in thickening and foam stability. Other useful suspending and thickening agents can be used instead of the alkanolamides such as sodium alginate; guar gum; xanthan gum; gum arabic; cellulose derivatives, such as methylcellulose, hydroxybutylcellulose, hydroxyethylcellulose, hydroxypropylcellulose and carboxymethylcellulose; and various synthetic polymeric thickeners, such as the polyacrylic acid derivatives. Suitable alkanolamides include, but are not limited to, those known in the art of hair care formulations, such as cocamide monoethanolamide (MEA), cocamide diethanolamide (DEA), soyamide DEA, lauramide DEA, oleamide monoisopropylamide (MIPA), stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA and combinations thereof. Other suitable suspending agents are disclosed in Oh et al. Patent No. 4,704,272; Grote et al. Patent No. 4,741,855; and Bolich, Jr. et al. Patent No. 4,788,006, which patents are hereby incorporated by reference.

Emulsion stabilizers also may be used in the mousse compositions of the present invention. Useful examples include, such compounds as polyethylene glycol, silicone copolyols, polyvinyl alcohol, sorbitan monostearate, oleth-2, sorbitan monolaurate, and nonionic block copolymers of ethylene oxide and propylene oxide such as those marketed by BASF Wyandotte under the name PLURONICS®. When present, such stabilizers comprise from about 0.05% to about 1%, preferably from about 0.1% to about 0.8%, by weight of the mousse composition.

The propellant gas included in the aerosol compositions of the present invention, in amounts of about 3% to about 50% by weight, can be any liquifiable and water-soluble gas. The preferred propellant is dimethyl ether (DME), used singly or admixed with other water-soluble gases, such as carbon dioxide, and/or nitrous oxide to obtain aerosols having reduced flammability.

The aerosol compositions also can contain a variety of other nonessential, optional components suitable for rendering such compositions more acceptable. Such conventional optional ingredients are well known to those skilled in the art, e.g., other emulsifiers such as anionics (e.g., sodium alkyl sulfate); preservatives such as benzyl alcohol, methyl paraben, propyl paraben and imidazolidinylurea; thickeners and viscosity modifiers such as a diethanolamide of a long chain fatty acid, fatty alcohols (i.e., cetearyl alcohol), sodium chloride, sodium sulfate, and ethyl alcohol; pH adjusting agents such as citric acid, succinic acid, sodium hydroxide and triethanolamine; coloring agents such as any of the FD&C or D&C dyes; perfume oils; chelating agents such as ethylenediaminetetraacetic acid; and, among many other agents, polymer plasticizing agents, such as glycols, glycerine, silicone-glycols, polyglycols, nonionic or anionic polymers, lubricants and penetrants such as volatile silicones and hydrocarbons, lanolin compounds, protein hydrolysates and other protein derivatives, emollients, perfumes. These optional materials are generally used individually at a level of from about 0.01% to about 10%, preferably from about 0.5% to about 5% by weight of the total composition.

The aqueous formulations of the present invention also can contain other conventional hair spray adjuvants in amounts which generally range from about 0.1% to about 2% by weight and preferably about 0.75% to about 1% by weight. Among the additives which can be used are penetrants, such as various lanolin compounds; protein hydrolysates and other protein derivatives; ethylene adducts and polyoxyethylene cholesterol; and the like.

The optional alcohol employed in the composition is an aliphatic straight or branched chain monohydric alcohol having 2 to about 4 carbon atoms. Isopropanol and especially ethanol are preferred. The concentration of the alcohol in the composition should be less than about 20% by weight, preferably 0% to about 30% by weight and more preferably about 5% to about 20% by weight. Some alcohol, in an amount of about 2% to about 10% by weight provides faster drying of the styling aid after application to the hair. The amount of alcohol, e.g., ethanol, can be chosen to avoid polymer precipitation, as shown in the phase diagrams of Figures 1 and 2.

## Claims

1. An aqueous aerosol hair spray composition comprising a partially or fully neutralized sulfonated polystyrene homopolymer or copolymer fixative resin in an amount of about 2% to about 20% by weight of the composition; water in the amount of about 30% to about 90% by weight of the composition; 0% to about 5% alcohol; and a liquifiable, water-soluble propellant gas in an amount of about 5% to about 50% by weight of the composition.

2. The composition of Claim 1, wherein the propellant comprises dimethyl ether.

3. The composition of Claim 1, wherein the fixative resin is a copolymer containing an ethylenically unsaturated vinyl monomer capable of copolymerization with styrene.

4. The composition of Claim 1 further including a conditioning agent in an amount of about 0.1% to about 10% by weight of the composition.

5. The composition of Claim 4, wherein the conditioning agent is water-insoluble and is selected from the group consisting of a silicone conditioning agent, a volatile hydrocarbon conditioning agent, and mixtures.

6. The composition of Claim 1, wherein the alcohol is a straight or branched chain monohydric alcohol having 2 to about 4 carbon atoms and is included in the composition in an amount of about 1% to about 30% by weight of the composition.

7. The composition of Claim 1, wherein the alcohol is included in an amount of about 5% to about 25% by weight of the composition.

8. An aqueous hair spray composition comprising a partially or fully neutralized sulfonated polystyrene homopolymer or copolymer fixative resin in an amount of about 2% to about 20% by weight of the composition; water in an amount of about 30% to about 80% by weight of the composition; 0% to about 30% alcohol; and a water-soluble, liquifiable propellant gas in an amount of about 5% to about 35% by weight of the composition.

9. The composition of Claim 8, wherein the propellant comprises dimethyl ether.

10. The composition of Claim 9, wherein the propellant comprises a mixture of dimethyl ether with carbon dioxide or nitrous oxide.

11. A method of fixing human hair in a desired configuration comprising applying a hair spray composition containing a partially or fully neutralized sulfonated polystyrene fixative resin to the hair, while said hair is in said desired configuration, to adhere adjacent hairs together, said composition comprising a partially or fully neutralized sulfonated polystyrene fixative resin in an amount of about 2% to about 20% by weight of the composition; water in an amount of about 30% to about 90% by weight of the composition; 0% to about 30% alcohol; and about 5% to about 35% by weight of a liquifiable, water-soluble propellant gas.

12. The method of Claim 11, wherein the propellant comprises dimethyl ether.

13. The method of Claim 12, wherein the propellant comprises a mixture of dimethyl ether with carbon dioxide or nitrous oxide

14. The method of Claim 11, wherein the fixative resin is a copolymer of a sulfonated polystyrene with a vinyl monomer.

15. The method of Claim 11, wherein the alcohol is a straight or branched chain monohydric alcohol having 2 to about 4 carbon atoms and is included in the composition in an amount of about 1% to about 30% by weight of the composition.

16. The method of Claim 15, wherein the alcohol is included in an amount of about 5% to about 25% by weight of the composition.

17. The hair spray composition of claim 1 wherein said alcohol comprises about 2% to about 5% of the composition.
